# EUROPEAN PATENT APPLICATION

(11) **EP 4 810 014 A1**
(43) Date of publication of application: **23.09.2026**
(21) Application number: 25382274.6
(22) Date of filing: 21.03.2025
(51) Int. Cl.: A61K 8/9789, A61Q 19/02

(54) **PROCESS FOR PREPARING A MYRTUS COMMUNIS HYDROLYSATE**

(71) Applicant: Bella Aurora Labs, S.A., 08690 Santa Coloma de Cervelló (Barcelona) (ES)
(72) Inventor: Hernández Navarro, Sergi, Santa Coloma de Cervelló (ES); Farré Quesada, Anna María, Santa Coloma de Cervelló (ES); Calvo Manso, Enrique, Reus (ES); Mulero Abellán, Miquel, Reus (ES); Bravo Vázquez, Francisca Isabel, Santa Marta de los Barros (ES); Muguerza Marquínez, Begoña, Tarragona (ES)
(74) Representative: Pons IP

(57) **Abstract**

The present invention relates to a process for preparing a *Myrtus communis* hydrolysate comprising the enzymatic hydrolysis of an aqueous extract of *Myrtus communis* successively with a cellulase and with a protease. The present invention also relates to a *Myrtus communis* hydrolysate that is obtainable with such process, to a cosmetic composition comprising the hydrolysate and to their use for skin whitening. The invention also relates to a method for skin whitening comprising topically applying said hydrolysate or the cosmetic composition comprising it.

## Description

### Technical field

The present invention relates to a process for preparing a *Myrtus communis* hydrolysate and to its use as skin whitening substance.

### State of the art

Melanin is a dark pigment which is responsible for skin colour and is synthesized in melanocytes, a type of cells found in the epidermis. The process to produce melanin is known as melanogenesis, and takes place within the melanosomes, which are vesicles inside the melanocytes. Two types of melanin are produced: brownish black eumelanin and reddish yellow pheomelanin.

The melanin generated in the melanocytes is transferred to neighbouring keratinocytes, thus the pigment becomes visible in the epidermis surface and becomes progressively darker due to photooxidation.

The concern about skin appearance, in particular, the desire of lighten the skin tone or improve skin complexion, has boosted the research into skin whitening cosmetic products. These products are aimed at inhibiting melanogenesis, in order to obtain a reduction of the physiological skin pigmentation.

A key enzyme of melanogenesis is tyrosinase, a glycoprotein located in the membrane of the melanosomes, which catalyses two steps of the synthesis of melanin from L-tyrosine. Other two enzymes involved in melanogenesis are tyrosinase-related protein 1 (Tyrp1) and tyrosinase-related protein 2 (Tyrp2).

However, melanogenesis is a complex process which is regulated by a series of multistep signal transduction cascades and is influenced by a variety of extrinsic and intrinsic factors.

The mechanisms involved in skin pigmentation and the main skin-whitening agents available are disclosed, for example, in Fiske et al., A systematic review of skin whitening product, Int. J. Pharm. Sci. Rev. Res., 2021, Article No. 13, 102-113, or in Kumari et al., Melanogenesis inhibitors, Acta Derm. Venereol., 2018, 98, 924-931.

The most common approach for the whitening and depigmentation of skin is to reduce the melanin production by means of tyrosinase inhibition. Among the known tyrosinase inhibitors useful for skin-lightening are, for example, hydroquinone and its derivatives arbutin and deoxyarbutin, which show inhibition of melanogenesis, though their use may cause melanocyte cytotoxicity. Kojic acid, which is a naturally occurring fungal metabolite obtained from several species of fungi, such as *Aspergillus, Acetobacter* and *Penicillium,* is successfully used for treating melasma, though it can cause several adverse effects, such as contact dermatitis, sensitization and erythema. Azelaic acid (nonanedioic acid) is another tyrosinase inhibitor also commonly used as depigmenting agent. Also, polyphenols such as resveratrol or isoflavones such as glabridin, have been reported to have skin-lightening effects through tyrosinase inhibition.

Another approach used for skin-lightening is the inhibition of the transfer of mature melanosomes containing melanin to the keratinocytes. Some skin-lightening substances which are believed to act through this mechanism are protease-activated receptor 2 (PAR-2) inhibitors, such as soymilk and soybean extracts, and niacinamide.

Antioxidants are also commonly used for skin lightening, as they can neutralize reactive oxygen species (ROS) in the skin (which activate melanogenesis) and can also reduce the direct photooxidation of pre-existing melanin. Among the antioxidants commonly used as skin-lightening agents are vitamin E, vitamin B, vitamin C and ascorbic acid derivatives, such as magnesium ascorbyl phosphate, ascorbyl palmitate, and ascorbyl glucoside, for example.

Many of the known skin-whitening substances are derived from plants; furthermore, several plant extracts have been used as skin whitening agents, for example, extracts of *Morus alba, Acacia bark, Glycyrrhiza glabra, Green tea, Hippophae rhamnoides* and *Cassia fistula,* among others (Khan et al., Whitening efficacy of plant extracts including Hippophae rhamnoides and Cassia fistula extracts on the skin of Asian patients with melasma, Postepy Dermatol. Alergol., 2013, 30(4), 226-32).

The patent application US-A-2011/0027205 discloses an alcoholic or hydroalcoholic extract of *Myrtus communis* as depigmenting agent.

Despite the substances and compositions for skin lightening described so far in the art, there is still the need for improved cosmetic products and new targets, capable of providing safe and effective depigmentation of the skin.

### Object of the invention

The object of the present invention is a process for preparing a *Myrtus communis* hydrolysate.

Another aspect of the invention is the *Myrtus communis* hydrolysate obtainable with said process.

Another aspect of the invention is the use of said hydrolysate for skin whitening.

Another aspect of the invention is a method for skin whitening comprising the step of topically applying said hydrolysate.

Another aspect of the invention is a cosmetic composition comprising said hydrolysate.

Another aspect of the invention is the use of said cosmetic composition for skin whitening.

Another aspect of the invention is a method for skin whitening comprising the step of topically applying said cosmetic composition.

### Description of the Drawings

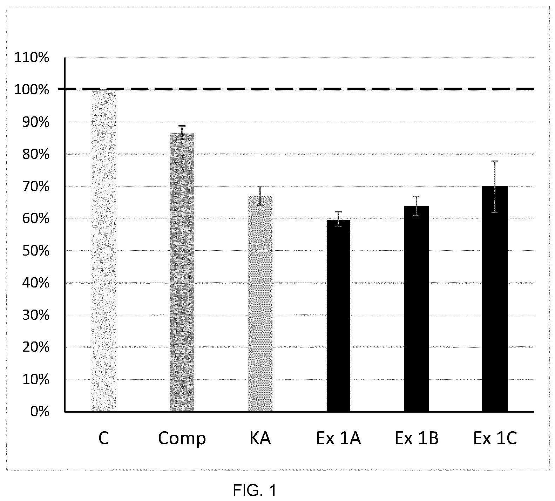

Figure 1 is a graph showing the results of the *in vitro* assay of Example 2, where the inhibition of extracellular melanin secretion was tested in B16F10 cells stimulated with melanocyte-stimulating hormone (α-MSH). The x-axis represents the different treatments: Comp *(M. communis* extract, not enzymatically hydrolysed of Comparative Example), KA (kojic acid) and Examples 1A, 1B and 1C (hydrolysates according to the invention). The y-axis represents the % of melanin secretion, relative to control (C, stimulated, non-treated cells), which is taken as 100%.

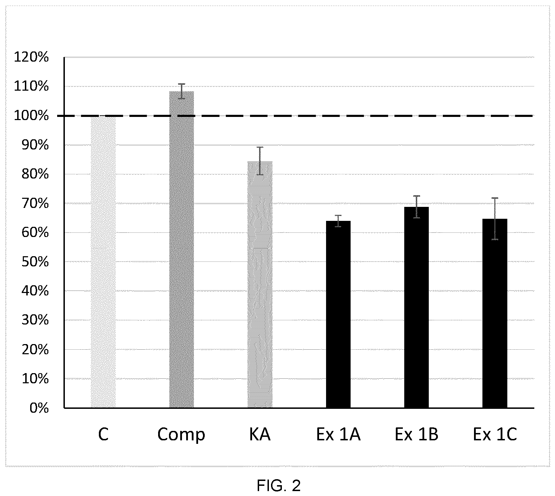

Figure 2 is a graph showing the results of the *in vitro* assay of Example 2, where the inhibition of intracellular melanin secretion was tested in B16F10 cells stimulated with melanocyte-stimulating hormone (α-MSH). The x-axis represents the different treatments: Comp *(M. communis* extract, not enzymatically hydrolysed of Comparative Example), KA (kojic acid) and Examples 1A, 1B and 1C (hydrolysates according to the invention). The y-axis represents the % of melanin secretion, relative to control (C, stimulated, non-treated cells), which is taken as 100%.

### Detailed description of the invention

The object of the present invention is a process for preparing of a *Myrtus communis* hydrolysate, wherein the process comprises the following steps:
a) extracting *Myrtus communis* material with an aqueous solvent;
b) enzymatically hydrolysing the extract obtained in step a) with a cellulase; and
c) enzymatically hydrolysing the hydrolysate obtained in step b) with a protease.

The authors of the present invention have developed a new skin-lightening substance, which is obtainable by a process comprising the enzymatic hydrolysis of *Myrtus communis,* which shows outstanding anti-melanogenic activity and is therefore suitable as cosmetic active ingredient for the preparation of effective skin-lightening compositions.

Along the present description, as well as in the claims, the singular expressions, generally preceded by the articles "a", "an" or "the", are meant to include also the plural forms, unless the context clearly indicates otherwise. Numeric values preceded by the term "about" are meant to include also a certain variation around such value, namely a variation or ±5% of the stated amount. Numeric ranges defined by lower and upper endpoints, using the expressions "from ... to...", or "between ... and ...", are meant to include also said stated endpoints and they also include any narrower sub-range.

A cosmetic active agent or cosmetic active ingredient is any substance intended to be applied on the body surface, particularly on the skin, hair or nails, to provide a cosmetic effect. A cosmetic effect relates to beautify and/or improve the feeling or sensory aspects of normal, nondiseased skin, hair or nail. Cosmetic effects do not involve any therapeutic effect, i.e., cosmetics are not intended to prevent or ameliorate any disease.

A cosmetic effect is, for example, the whitening of skin. The terms "whitening", "lightening", or "depigmentation" are used interchangeably herein and are referred to the process of lightening hyperpigmented skin, typically, to eliminate or reduce dark spots, blemishes or marks of the skin, including age spots, post-acne marks, uneven skin tone, post-inflammatory hyperpigmentation or melasma and lentigo spots, for example. In the present description, the terms "spots", "marks" and "blemishes" are meant to be equivalents and are used interchangeably.

### Myrtus communis

The process according to the present invention comprises the enzymatic hydrolysis of an aqueous extract of *Myrtus communis* material.

*Myrtus communis (M. communis),* commonly known as common myrtle or true myrtle, is a species of flowering plant in the myrtle family Myrtaceae. It is an evergreen shrub or tree up to 5 m, native to the Mediterranean region and southwest Europe, with up to 5 cm long leaves, which are egg-lanceshaped, smooth-edged, leathery and tapering to the tip. Flowers are white, up to 3 cm diameter, and the fruits are berries, which are blue-black when ripe (in: Tucker et al., The Encyclopedia of Herbs. A Comprensive Reference to Herbs and Flavor and Fragance, 2009, Timber Press, ISBN-13: 978-0-88192-994-2, p: 328-332).

The *Myrtus communis* material used in the process of the invention can be any part of the *Myrtus communis* plant.

Typically, the *Myrtus communis* material is selected from the leaves and the fruit of *Myrtus communis,* and mixtures thereof. In an embodiment, the *Myrtus communis* material are leaves of *Myrtus communis.*

### Process for preparing the hydrolysate

The process for preparing the *Myrtus communis* hydrolysate comprises three consecutive steps, namely:
a) extracting *Myrtus communis* material with an aqueous solvent;
b) enzymatically hydrolysing the extract obtained in step a) with a cellulase; and
c) enzymatically hydrolysing the hydrolysate obtained in step b) with a protease.

As is well-known in the art, a hydrolysate is a product obtained by hydrolysis. The hydrolysate of the present invention is a product obtained by enzymatic hydrolysis, i.e., using enzymes, which facilitate the cleavage of bonds.

Also as is well-known in the art, an extract is a product obtained by extracting a part of a raw material, typically using a solvent.

The *Myrtus communis* material, preferably leaves of *Myrtus communis,* is typically first ground to a fine powder. Any known method may be used for crushing the material, typically using a mill or a grinder, optionally previous freezing the plant material.

The solvent used for the extraction of step a) is an aqueous solvent, i.e., it comprises water. Preferably, the aqueous solvent comprises at least 60% of water, preferably at least 70% of water, still more preferably at least 80% of water, still more preferably at least 90% of water, still more preferably at least 95% water, and still more preferably the aqueous solvent is water.

When the solvent comprises another solvent mixed with water, said solvent is typically an alcohol, preferably ethanol.

Preferably, all the steps of the process are performed using solely water as solvent.

The aqueous extraction of step a) is performed conventionally. The plant, preferably the leaves, are typically grinded and mixed with water, to a concentration of from about 5% w/v to about 25% w/v, preferably from about 8% w/v to about 20% w/v, and more preferably from about 10% w/v to about 15% w/v. The mixture is generally heated, preferably to 90-100 °C, typically to about 100 °C, during a period of time generally of at least 15 minutes, for example from about 15 minutes to about 3 hours, preferably from about 15 minutes to about 2 hours, more preferably from about 15 minutes to about 1 hour, for example during about 30 minutes. Said aqueous extraction, is preferably performed under stirring, typically under mechanical stirring, for example at a speed comprised between 20-350 rpm.

The aqueous extract thus obtained is then subjected to enzymatic hydrolysis of step b). Typically, the aqueous mixture extract directly obtained in step a) is used for the next enzymatic hydrolysis, without separation of the aqueous solution from the insoluble material.

Step b) of the process for obtaining the hydrolysate of the invention includes the treatment with a cellulase.

Cellulases, as is well-known in the art, cleave beta-1,4-glycosidic bonds in cellulose. This enzyme class can be divided into endo-cellulases (cellulase, endoglucanase, EC 3.2.1.4) and exo-cellulases (cellulose 1,4-beta-cellobiosidase (nonreducing end), exoglucanase, cellobiohydrolase, E.C. 3.2.1.91). Endo-cellulase type cellulases catalyse the endo-hydrolysis of 1,4-beta-D-glucosidic linkages in cellulose, lichenin, and cereal beta-D-glucans. Exoglucanases hydrolyse the 1,4-beta-D-glucosidic linkages in cellulose and cellotetraose, releasing cellobiose from the nonreducing ends of the chains (see, for example, W. Aehle, Ed., Enzymes in Industry, 2007, Wiley-VCH Verlag, p: 173-176; or Expasy, Enzyme nomencalure database, Swiss Institute of Bioinformatics). Cellulases are produced chiefly by fungi, bacteria and protozoans. Cellulases of both types are suitable for the present invention.

In an embodiment of the invention, the cellulase is an endo-cellulase (EC 3.2.1.4).

In a particular embodiment, the cellulase is a cellulase from *Trichoderma reesei,* for example, the cellulase commercially available under the tradename Celluclast^{®} 1.5 L.

The amount of cellulase used for the enzymatic hydrolysis can be expressed as endo-glucanase units (EGU), where 1 EGU indicates the amount of cellulase required releasing 1 micromole of glucose per minute, under the assay conditions. Typically, from about 10 to about 20 EGUs per gram of *M. communis* material is used, preferably, about 15 EGUs/g.

The treatment with the cellulase is generally maintained for at least 15 minutes, for example, for from about 15 minutes to about 3 hours, preferably for from about 30 minutes to about 2 hours, for example, for about 1 hour. The temperature during the incubation is maintained in the range 25-65 °C, preferably in the range 40-60 °C, and more preferably at about 50 °C.

Preferably, the incubation with the cellulase is performed under stirring, typically under mechanical stirring, for example at a speed comprised between 50 and 350 rpm.

Step c) of the process comprises the treatment with a protease. The treatment with the protease is typically directly performed on the hydrolysate mixture obtained after step b), i.e., without any separation step of solids.

Different types of proteases are suitable to be used in this step. The protease is typically selected from an endopeptidase, an exopeptidase, and mixtures thereof. This means that the protease is typically either one or more endopeptidases, or one or more exopeptidases, or a mixture of one or more endopeptidases with one or more exopeptidases.

As is well known in the art, an endopeptidase (or endoproteinase) is a protease that hydrolyses peptide bonds of nonterminal amino acids, while an exopeptidase is a protease that hydrolyses the terminal (or the penultimate) peptide bond, releasing a single amino acid, dipeptide or a tripeptide from the peptide or protein chain. The exopeptidases can be classified as aminopeptidases or carboxypeptidases, depending on whether the amino acid is released from the amino or the carboxy terminal end, respectively.

The endopeptidase to be used in the process according to the present invention may be selected from a serine endopeptidase (EC 3.4.21), a cysteine endopeptidase (EC 3.4.22), an aspartic endopeptidase (EC 3.4.23), a metalloendopeptidase (EC 3.4.24), other endopeptidases of unknown catalytic mechanism (EC 3.4.99), and mixtures thereof.

In an embodiment, the endopeptidase is selected from a serine endopeptidase, a metalloendopeptidase, and mixtures thereof.

In a particular embodiment, the serine endopeptidase is a subtilisin (EC 3.4.21.62). Subtilisin, as is well-known in the art, is a serine endopeptidase, which can be obtained from *Bacillus subtilis* and from other *Bacillus* species. It is widely available from several commercial sources, for example, under the tradename Alcalase^{®} (Novozymes) that has primarily subtilisin A from *Bacillus licheniformis.*

In an embodiment, the serine endopeptidase is Alcalase^{®}.

In a particular embodiment, the serine endopeptidase is a prolyl oligopeptidase (EC 3.4.21.26), also known as post-proline cleaving enzyme, or post-proline endopeptidase or prolyl endopeptidase.

In a particular embodiment, the metalloendopeptidase is a *Bacillus* metalloendopeptidase (EC 3.4.24.28). *Bacillus* metalloendopeptidase is a well-known protease, which is also known as "bacillolysin", or "neutral protease", or *"Bacillus subtilis* neutral protease", or *"Bacillus subtilis* neutral proteinase". It is a metalloendopeptidase found, for example, in *Bacillus subtilis* or *Bacillus amyloliquefaciens.* It is widely available from several commercial sources, for example, under the tradename Neutrase^{®} (Novozymes), which is a zinc metalloendopeptidase from *Bacillus amyloliquefaciens,*

In an embodiment, the metalloendopeptidase is Neutrase^{®}.

The exopeptidase may be selected from an aminopeptidase (EC 3.4.11), an acylamino-acid hydrolase (3.4.12), a dipeptidase (EC 3.4.13), a dipeptidyl-peptidase or tripeptidyl-peptidase (EC 3.4.14), a peptidyl-dipeptidase (EC 3.4.15), a serine-type carboxypeptidase (EC 3.4.16), a metallocarboxypeptidase (EC 3.4.17), a cysteine-type carboxypeptidase (EC 34.18), an omega peptidase (EC 3.4.19), and mixtures thereof.

In an embodiment, the exopeptidase is selected from an aminopeptidase (EC.3.4.11), a dipeptidyl-peptidase (EC 3.4.14), and mixtures thereof.

A preferred aminopeptidase is a leucyl aminopeptidase (or leucine aminopeptidase, EC 3.4.11.1).

A preferred dipeptidyl-peptidase is dipeptidyl-peptidase IV (EC 3.4.14.5).

In a particular embodiment of the invention, the protease of step c) is a subtilisin (EC 3.4.21.62), and preferably is subtilisin A from *Bacillus licheniformis* (alcalase).

In a particular embodiment of the invention, the protease of step c) is a *Bacillus* metalloendopeptidase (EC 3.4.24.28).

In a particular embodiment of the invention, the protease of step c) is a mixture of a subtilisin (EC 3.4.21.62) and a *Bacillus* metalloendopeptidase (EC 3.4.24.28).

In a particular embodiment of the invention, the protease of step c) is a prolyl oligopeptidase (EC 3.4.21.26).

In a particular embodiment of the invention, the protease of step c) is a mixture of an endopeptidase and an exopeptidase, preferably wherein the exopeptidase comprises a leucyl aminopeptidase (EC 3.4.11.1).

Some commercially available enzymes are mixtures of different proteases, and can be suitable used in step c) of the process.

For example, Flavourzyme^{®} (Novozymes) is a well-known enzyme mixture comprising fungal exopeptidases and endopeptidases from *Aspergillus oryzae,* with high leucine aminopeptidase activity, that contains leucyl aminopeptidase and dipeptidyl-peptidase IV, as well as neutral protease and alkaline protease, among others (Merz et al., Flavourzyme, an Enzyme Preparation with Industrial Relevance: Automated Nine-Step Purification and Partial Characterization of Eight Enzymes, J. Agric. Food Chem., 2015, 63, 5682-93).

Protanex^{®} (Novozymes) is a mixture of bacillolysin (EC 3.4.24.28) and subtilisin (EC 3.4.21.62), from *Bacillus licheniformis* and *Bacillus amyloliquefaciens*

The amount of protease used in the hydrolysis, as is well known in the art, is typically expressed on the basis of its catalytic activity.

Anson Units (AUs) are commonly used for proteases, where one AU is defined as that amount of enzyme which under standard conditions digests haemoglobin at an initial rate that per minute liberates an amount of trichloroacetic acid-soluble product which gives the same colour with phenol reagent as one milli-equivalent of tyrosine.

For example, from about 0.05 to about 1.0 Anson Units (AUs) of protease per gram of protein in the substrate may be used, preferably, from about 0.1 to about 0.6 AU/g protein.

Alternatively, for prolyl endopeptidases, the amount of enzyme can be expressed as PPU (prolyl peptidase units or proline protease units), based on the release of p-nitroanilide (pNA) from the synthetic substrate Z-Gly-Pro-pNA (carbobenzoxy-glycyl-proline-p-nitroanilide) at pH 4.6 and 37°C, and wherein one PPU is defined as the amount of enzyme required to release one micromole of p-nitroanilide (pNA) from Z-Gly-pro-pNA in 1 min under conditions of the assay (pH 4.6, 37.0°C).

For example, from about 0.1 to about 2 Proline Protease Units (PPUs) of protease per gram of protein in the substrate may be used, preferably, from about 0.2 to about 1.5 PPU/g protein.

Alternatively, when the exopeptidase comprises a leucine aminopeptidase, it can be expressed as Leucine Aminopeptidase Units (LAPU) per gram of substrate, wherein one LAPU is defined as the amount of enzyme causing the hydrolysis of 1 µmol *L*-Leucine-*p*-nitroanilide to *L*-Leucine and *p*-nitroaniline per minute.

For example, from about 10 to about 150 Leucine Aminopeptidase Units (LAPUs) per gram of protein in the substrate may be used, preferably, from about 15 to about 60 LAPU/g protein.

On this basis, the skilled in the art will not have difficulties in suitably adjusting the amount of protease to perform the hydrolysis in step c).

To this end, the amount of protein in the substrate can be easily determined using known methods, typically using the Kjeldahl method.

The treatment with the protease is maintained for a period of time typically of at least 15 minutes, for example, ranging from about 15 minutes to about 8 hours, preferably ranging from about 30 minutes to about 4 hours, and more preferably ranging from about 1 hour to about 2 hours, for example, for about 1 hour. The temperature during the incubation is generally maintained in the range 25-65 °C, preferably 40-60 °C, and more preferably at about 50 °C.

Preferably, the incubation with the protease is performed under stirring, typically under mechanical stirring, for example at a speed comprised between 50 and 350 rpm.

After step c) the hydrolysate is generally collected as an aqueous solution, by removal of the non-soluble material, typically by decantation, centrifugation and/or filtration.

The obtained hydrolysate aqueous solution may be used as such, or may be further concentrated/diluted, or may be dried.

The aqueous hydrolysate can be dried using different techniques, for example, by freeze-drying, spray-drying or by heating, to obtain a dry hydrolysate.

A "dry hydrolysate", as used herein, means that the water content of the hydrolysate is typically less than about 10% (w/w), preferably less than about 7% (w/w), more preferably less than about 5% (w/w), still more preferably less than about 4% (w/w), still more preferably less than about 3% (w/w), still more preferably less than 2% (w/w) and still more preferably less that about 1% (w/w).

Another aspect of the invention is the *Myrtus communis* hydrolysate obtainable with this process.

### Anti-melagonesis activity of the hydrolysate of M. communis

As shown in the *in vitro* assay of Example 2, it was surprisingly found that the *M. communis* hydrolysate according to the present invention is able to inhibit melanogenesis, both in extracellular and intracellular melanin, with reductions of 30-40% and 31-36% respectively.

Notably, the anti-melanogenic activity of hydrolysates according to the invention is superior to that of kojic acid, which is a recognized anti-melanogenic substance. In the extracellular melanin assay, for example, the hydrolysates showed comparable inhibition to kojic acid, but in the intracellular melanin assay, the inhibition was 30-36% *vs.* only 16% for kojic acid.

On the other hand, it was also found that the melanin inhibitory activity of the *M*. *communis* hydrolysate according to the invention was substantially greater than that of a simple extract of *M. communis* not subjected to any enzymatic hydrolysis. Indeed, in the extracellular melanin assay, the *M. communis* extract (Comp) showed only 13% melanin inhibition, vs. 30-40% for the hydrolysates of the invention. Furthermore, in the intracellular melanin assay, the extract (Comp) even caused a light increase of melanin *(8%), vs.* 31-36% decrease for the hydrolysates of the invention.

In the BACE2 inhibitory assay of Example 4, it was found that the melanin inhibitory activity of the hydrolysate of the invention appears to be linked to an inhibition of this enzyme. BACE2 (beta-secretase 2, or Memapsin-1, EC 3.4.23.45) belongs to the family of transmembrane aspartic proteases. Among other biological functions, BACE2 inhibition can cause loss of pigmentation, as BACE2 cleaves the integral membrane form of PMEL within the juxtamembrane domain and exerts its role in melanosome biogenesis (Yan R., Physiological Functions of the β-Site Amyloid Precursor Protein Cleaving Enzyme 1 and 2., Front. Mol. Neurosci., 2017, 10:97).

According to these experimental results, the *Myrtus communis* hydrolysate according to the invention is particularly useful for cosmetic use as skin-whitening agent.

Another aspect of the invention is, therefore, the use of the hydrolysate of the invention for skin whitening. It is understood that said use is a cosmetic use (non-therapeutic).

Another aspect of the invention is a method for skin whitening comprising the step of topically applying a cosmetically effective amount of the hydrolysate of the invention to a subject in need thereof.

### Cosmetic compositions

The hydrolysate of the present invention is suitable to be incorporated into skin whitening cosmetic compositions.

Another aspect of the present invention is, therefore, a cosmetic composition comprising the hydrolysate of the invention.

The cosmetic composition typically comprises the *M. communis* hydrolysate of the invention, as active cosmetic ingredient, optionally in combination with other cosmetic active ingredients, and at least one dermatologically acceptable carrier or vehicle. A substance is considered to be "dermatologically acceptable" or "cosmetically acceptable" if it is suitable and non-toxic for use in contact with human skin tissue.

The composition may be in the form of cream, gel, cream-gel, lotion, paste, foam, solution, suspension, emulsion, milk, or stick preparation, for example.

Suitable carriers may be, for example, anhydrous, as mixtures of fats, waxes, animal and plant oils and solid and liquid hydrocarbons. Or the carrier may be water or an aqueous solution of hydrophilic substances. Preferably, the carrier is in the form of an emulsion. Emulsions may be, typically, oil-in-water emulsions, water-in-oil emulsions, water-in-oil-in-water, oil-in-water-in-oil or water-in-silicone emulsions. An emulsion may generally be described as having a continuous aqueous phase (oil-in-water and water-in-oil-in-water) or a continuous oil phase (water-in-oil and oil-in-water-in-oil). The oil phase may comprise silicone oils, non-silicone oils such as paraffin hydrocarbons, fatty alcohols, fatty acids, fatty acid esters, waxes or plant oils, or mixtures thereof. The aqueous phase may comprise water or a water solution of hydrophilic substances, such as polyols, alpha hydroxy acids, amino acids, protein hydrolysates, simple sugars, and polysaccharides.

Emulsifiers, which are common components of emulsions, are surface-active agents (surfactants) and include non-ionic surfactants, anionic surfactants, cationic surfactants and amphoteric surfactants. Non-ionic surfactants include, among others, ethoxylated fatty alcohols, ethoxylated fatty acid esters, alkyl glucosides or alkyl oligoglucosides, ethoxylated sorbitan fatty acid esters, monoglycerol/polyglycerol fatty acid esters, ethoxylated glycerin monesters, ethoxylated polyglyceryl esters, alkyl dimethylamine oxides, or poloxamers, among others. Anionic surfactants include alkaline soaps, alkyl sulphates, alkyl ether sulphates, alkyl sulphosuccinates, acyl sarcosinates or acyl isethionates, among others. Cationic surfactants include quaternary ammonium salts, or pyridine salts, among others. Amphoteric surfactants include imidazoline betaine derivatives, alkylbetaines, amidobetaines and sulphobetaines.

Other common ingredients in the formulation are, for example, emollients, humectants, preservatives, viscosity controlling agents, antioxidants, pH regulators, UV filters, chelating agents, perfumes and colorants. Common emollients are, for example, paraffin hydrocarbons, silicones, fatty alcohols, fatty acids, esters of fatty acids with alcohols, triglycerides, ceramides, phospholipids and waxes. Humectants include polyhydroxy alcohols, proteins and hydroxyl acids. Common preservatives include sorbic acid and its salts, benzoic acid and its salts, parabens, imidazolidinyl urea, diazolidinyl urea, DMDM hydantoin, sodium hydroxymethylglycinate, methylchloroisothiazolinone/methylisothiazolinone, benzyl alcohol and 2-phenoxyethanol, among others. Other additives may also be added for controlling the viscosity of the formulation, for example, xanthan gum, gellan gum, carrageenans, pectin, starch derivatives, carbomers, cellulose derivatives (hydroxyethylcellulose, hydroxypropylcellulose, hydroxypropyl methylcellulose, methylcellulose, ethylcellulose, or carboxymethylcellulose, for example), polyamides, glutamides, colloidal silica or waxes (e.g., beeswax or vegetable waxes), among others.

The cosmetic composition of the invention has typically a slightly acidic pH, close to the physiological pH of the skin. Common acidity regulators are organic acids, including hydroxy acids and fatty acids. Some of the most common pH regulators in cosmetic emulsions are hydroxy acids such as lactic and citric acids.

The above cited ingredients, as well as many others suitable cosmetic formulation excipients, are well-known to the skilled in cosmetic formulation. Such cosmetic ingredients are commercially available from several companies, such as Comercial Química Massó, SA, Evonik, DuPont or Dow Corning, among others.

The preparation of the cosmetic composition is made according to procedures well-known to the skilled in cosmetic formulation, generally involving simple steps of mixing, and optionally heating the component ingredients.

The description of the main cosmetic ingredients and procedures may be found, for example, in: Cosmetic Formulation. Principles and Practice, Benson H.A.E., Roberts M.S., Rodrigues Leite-Silva V. and Walters K.A., editors, CRC Press, 2019, or in similar reference books. Also, regulated cosmetic substances and ingredients are disclosed in the European Commission database "Cosing" (https://ec.europa.eu/growth/tools-databases/cosing).

The amount of the *M. communis* hydrolysate in the composition typically ranges from about 0.001 mg to about 5 mg of hydrolysate per g of composition, preferably from about 0.01 mg to about 1 mg of hydrolysate per g of composition, more preferably from about 0.02 mg to about 0.5 mg of hydrolysate per g of composition, and still more preferably about 0.1 mg of hydrolysate per g of composition. The amount of hydrolysate in the composition is expressed as amount of dry hydrolysate, irrespectively of whether it is added to the composition as dry hydrolysate or as a solution.

The *M. communis* hydrolysate can be either the only whitening active substance of the composition, or it can be used in combination with other active ingredients.

In an embodiment, the cosmetic composition comprises an additional skin-whitening active ingredient.

Any known skin-whitening active disclosed in the state of the art, either of natural or synthetic origin, and acting through any whitening mechanism may be suitable to be included in the composition as additional skin-whitening agent. Among the disclosed mechanisms for skin lightening are tyrosinase inhibition, MITF inhibition, inhibition of the transfer of melanin to keratinocytes or antioxidative mechanisms, among others.

For example, the skin-whitening substances disclosed in the following review articles can be used as additional ingredients of the composition: Gillbro et al. The melanogenesis and mechanism of skin-lightening agents - existing and new approaches, Int. J. Cosm. Sci., 2011, 33, 210-221.; Kumari *et al. op. cit.;* or Zolghadri et al., A comprehensive review on tyrosinase inhibitors, J. Enzyme Inhib. Med. Chem., 2019, 34 (1), 279-309.

Some non-limiting examples of skin-whitening substances, suitable to be used in combination with the hydrolysate of the present invention, are cited hereinbelow. Among the substances with tyrosinase inhibitory activity, as is well-known in the art, are phenolic compounds such as hydroquinone, arbutin, deoxyarbutin, 4-(6-hydroxy-2-naphthyl)-1,3-bezendiol, resorcinol, 4-butylresorcinol, vanillin and its derivatives, 10'(Z)-heptadecenylhydroquinone, isotachioside and its glycoside derivatives. Also some flavonoid derivatives, mostly found in herbal plants, fruits and synthetic sources, are well-known as potent inhibitors of tyrosinase, belonging to the flavones (such as luteolin, apigenin, baicalein, chrysin, apigetrin, vitexin, baicalin, nobiletin, morusone or tangeretin, among others), flavonols (such as myricetin, kaempferol, quercetin, morin, isorhamnetin, galangin, rutin, quercitrin, or astragalin, among others), isoflavones (such as daidzein, genistein, glycitein, formononetin, genistin or daidzin, for example, some of them typically derived from soybean; or glabridin, isolated from the root of *Glycyrrhiza glabra;* among others), flavanones (such as naringenin, hesperetin, eriodictyol, naringin, hesperidin, or liquiritin, among others), flavanonols (such as taxifolin), flavanols (such as catechin, epicatechin, epi-gallocatechin, peicatechin gallate, epigallocatechin gallate or proanthocyanidins), anthocyanidins (such as cyanidin, delphinidin, malvidin, peonidin, or pelargonidin, among others), curcuminoids (curcumin or desmethoxycurcumin, for example), coumarins, chalcones (such as isoliquiritigenin, glabrene, 2,4,2',4'-hydroxycalcone, among others) or aurones (such as 4,6,4'-trihydroxyaurone, among others) classes. Other polyphenols with tyrosinase inhibiting activity are, for example, resveratrol and oxyresveratrol. Other substances belonging to different chemical classes have been disclosed as tyrosinase inhibitors, such as some terpenes (for example, bakuchiol or some carvacrol derivatives), quinones (such as aloin, and aloesin, found in *Aloe vera,* or tanshinone), pyridine derivatives (such as ((S)-(5-(benzyloxy)-1-octyl-4-oxo-1,4-dihydropyridin-2-yl)methyl 2-amino-3-phenylpropanoate), retinoids (such as adapalene and tretinoin, among others), carboxylic acids (such as azelaic acid or cinnamic acid, for example), azoles or thiazolidine derivatives.

Some of the known tyrosinase inhibitors may be of synthetic origin, while most of them are derived from plants, available as plant extracts, or are produced by fungi or bacteria. For example, kojic acid is a naturally occurring fungal metabolite obtained from species of *Acetobacter, Aspergillus* and *Penicillium.* Furthermore, a huge amount of plant species has been disclosed in the art to contain tyrosinase inhibiting substances.

Antioxidants are also commonly used for skin lightening, as they can neutralize reactive oxygen species (ROS) in the skin (which activate melanogenesis) and can also reduce the direct photooxidation of pre-existing melanin. Among the antioxidants commonly used as skin-lightening agents are vitamin E, vitamin B, vitamin C and ascorbic acid derivatives, such as magnesium ascorbyl phosphate, ascorbyl palmitate, and ascorbyl glucoside, for example.

Additionally, besides skin-whitening agents, the hydrolysate of the invention can also be used in combination with other active cosmetic ingredients, for example, antioxidants, astringents, anti-wrinkle agents, among others, if a combined cosmetic effect is desired.

Another aspect of the invention is the use of the cosmetic composition for skin whitening. It is understood that said use is a cosmetic use (non-therapeutic).

Another aspect of the invention is a method for skin whitening comprising the step of topically applying a cosmetically effective amount of said composition to a subject in need thereof.

Specific uses included in the skin whitening effect are the elimination or reduction of hyperpigmented marks on the skin (spots, blemishes), the smoothing uneven skin tone, or the lightening of the skin tone. The reduction of the marks may mean the reduction of the number of marks and/or the reduction of the size of the marks and/or lightening the intensity of the colour of the marks. Said hyperpigmented marks may be, for example, UV exposure related, post-scar marks, post-inflammation marks, melasma, lentigo or age-related marks, among others. The treated marks may be in any part of the body skin, preferably in the face, neck, arms and hands.

The skin-whitening use, as understood in the present invention, is exclusively cosmetic, related to beautify and/or improve the feeling or sensory aspects of normal, non-diseased skin, in particular, intended to the removal of non-pathologic marks and not intended to prevent or ameliorate any disease.

Among the hyperpigmented marks that can be treated with the hydrolysate of the invention and with the cosmetic composition comprising it are, for example, lentigo and melasma.

Lentigines (lentigo in singular), for example, are asymptomatic small sharply circumscribed brown macules. They are commonly due to chronic sun exposure (solar lentigo, also called liver spots) and occur most frequently on the sun-exposed areas, particularly on the face, neck and back of the hands. They typically first appear during middle age and increase in number with age. One of the causes of solar lentigo is the aggregation of aged-cells containing a dark pigment called lipofuscin, resulting in the formation of dark spots. Lipofuscin is the product of the oxidation of lipids and proteins, which may be triggered by the UV radiation.

Melasma (also known as chloasma), for example, is the formation of irregular-shaped dark brown spots or patches of pigmentation on the face and other sun-exposed areas of the body. Melasma patches are asymptomatic and are only of cosmetic concern. Melasma is thought to be caused by sun exposure, genetic predisposition and hormone changes, and is particularly common in women, especially in pregnant women.

A "cosmetically effective" amount means the necessary amount of the composition (or of the hydrolysate) to achieve the desired whitening effect. A skilled in the art can easily determine the necessary amount of the composition (or of the hydrolysate) to be used in each administration.

The compositions comprising the skin-whitening hydrolysate, according to the present invention, may be applied topically once, twice, or more times daily. The composition is typically applied by spreading it over the skin, generally, only to over the skin area to be depigmented. The duration of the treatment may be easily adjusted, for example, according to the intended effect, or according to the type and intensity of the marks to be removed. Typically, the treatment may be maintained for several days (e.g., 5-10 days), several weeks (e.g., 1-6 weeks), or months (e.g., 1-12 months).

The present invention can be defined according to the following embodiments:
1.- Process for preparing a *Myrtus communis* hydrolysate, wherein the process comprises the following steps:
   a) extracting *Myrtus communis* material with an aqueous solvent;
   b) enzymatically hydrolysing the extract obtained in step a) with a cellulase; and
   c) enzymatically hydrolysing the hydrolysate obtained in step b) with a protease.
2.- Process according to embodiment 1, wherein *Myrtus communis* material is selected from the leaves, the fruit and mixtures thereof, and preferably the *Myrtus communis* material are the leaves of *Myrtus communis.*
3.- Process according to embodiments 1 or 2, wherein the extraction of step a) is performed at a temperature ranging from 90 °C to 100 °C, preferably at about 100 °C, during a period of time or at least 15 minutes, preferably ranging from about 15 minutes to about 2 h.
4.- Process according to any one of embodiments 1 to 3, wherein the concentration of *M. communis* material in the aqueous solvent in the extraction step a) is from about 5% w/v to about 25% w/v, preferably from about 8% w/v to about 20% w/v, and more preferably from about 10% w/v to about 15% w/v.
5.- Process according to any one of embodiments 1 to 4, wherein the aqueous solvent of step a) comprises at least 60% of water, preferably at least 70% of water, still more preferably at least 80% of water, still more preferably at least 90% of water, still more preferably at least 95% water, and still more preferably the aqueous solvent is water.
6.- Process according to any one of embodiments 1 to 5, wherein the cellulase in step b) is an endo-cellulase (EC 3.2.1.4).
7.- Process according to any one of embodiments 1 to 6, wherein the enzymatic hydrolysis of step b) is performed at a temperature comprised in the range 25-65 °C, preferably in the range 40-60 °C, and more preferably at about 50 °C.
8.- Process according to any one of embodiments 1 to 7, wherein the incubation time with the cellulase in step b) is maintained for at least 15 minutes, for example, for from about 15 minutes to about 3 hours, preferably for from about 30 minutes to about 2 hours, for example, for about 1 hour.
9.- Process according to any one of embodiments 1 to 8, wherein the protease of step c) is selected from an endopeptidase, an exopeptidase, and mixtures thereof.
10.- Process according to embodiment 9, wherein the endopeptidase is selected from a serine endopeptidase (EC 3.4.21), a metalloendopeptidase (EC 3.4.24), and mixtures thereof.
11.- Process according to embodiment 10, wherein the serine endopeptidase is selected from a subtilisin (EC 3.4.21.62), a prolyl oligopeptidase (EC 3.4.21.26), and mixtures thereof.
12.- Process according to embodiments 10 or 11, wherein the metalloendopeptidase is a *Bacillus* metalloendopeptidase (EC 3.4.24.28).
13.- Process according to any one of embodiments 9 to 12, wherein the exopeptidase is selected from an aminopeptidase (EC 3.4.11), an acylamino-acid hydrolase (3.4.12), a dipeptidase (EC 3.4.13), a dipeptidyl-peptidase or tripeptidyl-peptidase (EC 3.4.14), a peptidyl-dipeptidase (EC 3.4.15), a serine-type carboxypeptidase (EC 3.4.16), a metallocarboxypeptidase (EC 3.4.17), a cysteine-type carboxypeptidase (EC 34.18), an omega peptidase (EC 3.4.19), and mixtures thereof, preferably is selected from an aminopeptidase (EC.3.4.11), a dipeptidyl-peptidase (EC 3.4.14), and mixtures thereof, and still more preferably is selected from a leucyl aminopeptidase (EC 3.4.11.1), dipeptidyl-peptidase IV (EC 3.4.14.5), and mixtures thereof.
14.- Process according to any one of embodiments 1 to 9, wherein the protease of step c) is a subtilisin (EC 3.4.21.62), and preferably is subtilisin A from *Bacillus licheniformis* (alcalase).
15.- Process according to any one of embodiments 1 to 9, wherein the protease of step c) is a *Bacillus* metalloendopeptidase (EC 3.4.24.28).
16.- Process according to any one of embodiments 1 to 9, wherein the protease of step c) is a mixture of a subtilisin (EC 3.4.21.62) and a *Bacillus* metalloendopeptidase (EC 3.4.24.28).
17.- Process according to any one of embodiments 1 to 9, wherein the protease of step c) is a prolyl oligopeptidase (EC 3.4.21.26).
18.- Process according to embodiment 9, wherein the protease is a mixture of an endopeptidase and an exopeptidase, preferably wherein the exopeptidase comprises a leucyl aminopeptidase (EC 3.4.11.1).
19.- Process according to any one of embodiments 1 to 18, wherein the enzymatic hydrolysis of step c) is performed at a temperature comprised in the range 25-65 °C, preferably in the range 40-60 °C, and more preferably at about 50 °C.
20.- Process according to any one of embodiments 1 to 19, wherein the incubation time with the protease in step c) is maintained for at least 15 minutes, for example, for from about 15 minutes to about 8 hours, preferably for from about 30 minutes to about 4 hours, and more preferably for from about 1 hour to about 2 hours, for example, for about 1 hour.
21.- Process according to any one of embodiments 1 to 20, wherein after step c) the obtained hydrolysate is separated from insoluble material and collected as an aqueous solution.
22.- Process according to embodiment 21, wherein the hydrolysate aqueous solution is dried to provide a dry hydrolysate.
*23.- Myrtus communis* hydrolysate obtainable by the process according to any one of embodiments 1 to 22.
24.- Use of the hydrolysate according to embodiment 23 for skin whitening.
25.- Method for skin whitening comprising the step of topically applying a cosmetically effective amount of the hydrolysate according to embodiment 23 to a subject in need thereof.
26.- Use according to embodiment 24 or method according to embodiment 25 for the elimination or reduction of hyperpigmented marks on the skin, the smoothing uneven skin tone, and/or the lightening of the skin tone.
27.- Cosmetic composition comprising the hydrolysate according to embodiment 23, optionally in combination with another cosmetic active ingredient, and at least one dermatologically acceptable carrier or vehicle.
28.- Cosmetic composition according to embodiment 27, wherein the composition is selected from the group consisting of a cream, gel, cream-gel, lotion, paste, foam, solution, suspension, emulsion, milk, and stick preparation.
29.- Cosmetic composition according to embodiments 27 or 28, wherein the composition comprises from about 0.001 mg to about 5 mg of the hydrolysate according to embodiment 23 per g of the composition.
30.- Cosmetic composition according to embodiment 29, wherein the composition comprises from about 0.01 mg to about 1 mg, preferably from about 0.02 mg to about 0.5 mg, and still more preferably about 0.1 mg of the hydrolysate according to embodiment 23 per g of composition.
31.- Cosmetic composition according to any one of embodiments 27 to 30, wherein the composition comprises an additional skin-whitening active ingredient.
32.- Use of the cosmetic composition according to anyone of embodiments 27 to 31 for skin whitening.
33.- Method for skin whitening comprising the step of topically applying a cosmetically effective amount of the composition according to any one of embodiments 27 to 31 to a subject in need thereof.
34.- Use according to embodiment 32 or method according to embodiment 33 for the elimination or reduction of hyperpigmented marks on the skin, the smoothing uneven skin tone, and/or the lightening of the skin tone.

### Examples

### Comparative Example Preparation of M. communis aqueous extract

An aqueous extract of *M. communis* was prepared, without including any enzymatic hydrolysis step.

To this end, the crushed plant material was first treated with water, in the same way as described herein below in Example 1 (Milli-Q water, 13.3%, w/v, at 100 °C for 30 minutes under stirring at 250 rpm). The mixture was allowed to cool down to 50 °C and, subsequently, the mixture was subjected to the same heating periods as in Example 1, but only with water, without adding any enzyme. Namely, the aqueous extract mixture was diluted and maintained at 50 °C during 2 h, under stirring at 250 rpm. The aqueous mixture was then centrifuged, the solution was filtered and freeze dried.

### Example 1 Preparation of M. communis hydrolysate

For preparing the hydrolysate according to the invention, leaves of *Myrtus communis* were used as raw material. Their protein content was calculated using the Kjeldahl method (7.97 ± 0.16 % (w/w)).

The leaves were mechanically crushed and mixed with Milli-Q water (13.3%, w/v). The mixture was heated at 100 °C with agitation at 250 rpm for 30 minutes. The mixture was allowed to cool down to 50 °C.

The aqueous extract mixture obtained, including the insoluble material, was incubated with the enzymes: first with the cellulase (Celluclast^{®} 1.5L (Novozymes) at 15 EGU/g of product) for 1 h at 50 °C under stirring at 250 rpm and, subsequently with the protease for 1 h at 50 °C under stirring at 250 rpm.

Three different proteases were used, in three different hydrolysates according to the invention:

| | |
|---|---|
| Example 1A | Alcalase^{®} (Novozymes) at 0.15 AU/g protein |
| Example 1B | Protamex^{®} (Novozymes) at 0.15 AU/g protein |
| Example 1C | Brewers Clarex^{®} (DSM-Firmenich) at 0.375 PPU/g protein |

Alcalase^{®} (Novozymes) has primarily subtilisin A (EC 3.4.21.62) from *Bacillus licheniformis.* Protamex^{®} (Novozymes) is a mixture of bacillolysin (EC 3.4.24.28) and subtilisin (EC 3.4.21.62), from *Bacillus licheniformis* and *Bacillus amyloliquefaciens.* Brewers Clarex^{®}, contains proline-specific endopeptidase (EC 3.4.21.26) derived from a selected strain of *Aspergillus niger.*

The obtained hydrolysates were first centrifuged at 3500 × g for 10 min and the supernatant was further filtered and freeze dried to obtain dry hydrolysates.

### Example 2 In vitro testing of the anti-melanogenic activity of the hydrolysates of Example 1

To evaluate anti-melanogenic activity of the hydrolysates, *in vitro* studies were conducted using B16F10 cells (ATCC CRL-6475), a murine melanoma-derived cell line. The cells were maintained in complete DMEM medium (Dulbecco's Modified Eagle's Medium) at 37°C in a humidified incubator with 5% CO₂ atmosphere.

For the experiments, B16F10 cells were seeded at a concentration of 8 × 10⁴ cells per well in 12-well culture plates. After 24 hours, the medium was replaced with complete DMEM without phenol red, and melanin secretion was stimulated by adding melanocyte-stimulating hormone (α-MSH) at a final concentration of 300 nM. Simultaneously, solutions of the compounds under study and the corresponding controls were added (final concentration 100 µg of dried product/mL).

Namely, the following substances were tested: hydrolysate of Examples 1A, 1B and 1C (100 µg/mL aqueous solution), Comparative Example (100 µg/mL aqueous solution), and Kojic acid (1 mM aqueous solution). Also, positive control cells, corresponding to melanin stimulated cells (α-MSH treated), without any anti-melanogenic treatment, and negative control cells, corresponding to cells without α-MSH and antimelanogenic treatments, were tested.

After 72 hours, the extracellular melanin (melanin secreted into the culture medium) was determined by measuring the optical density of 200 µL of the supernatant at a wavelength of 490 nm.

Subsequently, the intracellular melanin content was determined. To this end, the cells were harvested using RIPA buffer supplemented with protease and phosphatase inhibitors. After centrifugation, the supernatant was collected for protein quantification and protein expression analysis. The pellet was diluted with 300 µL of 1 M NaOH and incubated at 80 °C for 1 h. The absorbance of the diluted pellet at 400 nm was measured to determine intracellular melanin content.

The results for extracellular and intracellular melanin content were referenced to the result of the untreated cells (Control) and normalized to the amount of protein in each sample. Control cells were assigned to 100% of melanin production, and the reduction of melanin production for the assayed substances was referenced to the value of Control. The experiments were performed in triplicate or quadruplicate and the mean value and the standard deviation was calculated.

The results for the hydrolysates of Example 1 and comparative samples (extract of Comparative Example and kojic acid) are shown in the following table:

| | **Extracellular melanin** | | **Intracellular melanin** | |
|---|---|---|---|---|
| **Active** | *% vs. control mean value (SD)* | *% reduction vs. control* | *% vs. control mean value (SD)* | *% reduction vs. control* |
| Example 1A | 59.75 (2.26) | 40 | 64.00 (1.88) | 36 |
| Example 1B | 63.87 (2.99) | 36 | 68.83 (3.69) | 31 |
| Example 1C | 69.83 (8.00) | 30 | 64.76 (7.06) | 35 |
| Comparative Example | 86.63 (2.12) | 13 | 108.27 (2.51) | -8 |
| Kojic acid | 67.0 (3.03) | 33 | 84,45 (4.68) | 16 |

The results show reductions of 30-40% and 31-36% in extracellular and intracellular melanin, respectively for the hydrolysates according to the present invention, compared to untreated cells.

For Kojic acid, the reduction in extracellular melanin was comparable (33%), while the reduction in intracellular melanin was inferior (16%) in comparison with the hydrolysates of the invention.

The extract of Comparative Example was clearly inferior for the reduction of both intracellular and extracellular melanin. The extracellular melanin was only reduced in 13% versus control, while intracellular melanin was even increased in about 8%.

The reduction of melanin *vs*. control (C), taken as 100% melanin content, is represented in Figures 1 and 2. The results of extracellular melanin are shown in Figure 1 and the results of the intracellular melanin are shown in Figure 2.

### Example 3 In vitro testing of anti-melanogenesis in artificial skin

The anti-melanogenesis activity of the hydrolysate according to the invention was also assessed using the reconstructed human pigmented epidermis (RHPE) *in vitro* model, as disclosed, for example in the article Sahuc F, Reconstructed human pigmented epidermis (rhpe): an in vitro model for the evaluation of melanogenesis, SOFW J., 2009, 135 (7).

This is a 3D model providing a structure that reproduces a histological morphology comparable to the *in vivo* human tissue, consisting of multi-layered, stratified and pigmented epidermis. The reconstructed human pigmented epidermal tissue is composed of normal human keratinocytes cultivated in the presence of melanocytes (which can be of 3 different phototypes) localized in the basal layer. The different tanning degrees of these constructs correspond macroscopically to 3 different phototypes of human skin.

They are cultured on a support that makes them in contact with the culture medium at the bottom, and in contact with the air at the top, where the products are applied in a similar way to how it would be done to skin.

The test kit employed was SkinEthic^{™} RHPE/Pigmented Epidermis (RHPE/S/10/IV: phototype IV, 0.5 cm², day 10 of culture) obtained from the company Episkin.

The test is based on the quantification of the reduction of melanin content after daily topical application of the tested product for 5 days. The models are then dissolved to measure the melanin generated by measuring the absorbance of the solutions. The reduction degree in melanin content achieved for each tested product is related to its depigmenting strength.

In general, three RHPE was used for each tested product for measuring melanin amount, and the mean value was then calculated.

The tested compositions were prepared with the ingredients listed in the following table:

| **Ingredients** | | **Weight** % |
|---|---|---|
| A | Deionized water | 75,15 |
| A | Dermosoft^{®} OMP (Methylpropanediol, Caprylyl Glycol and phenylpropanol) | 2,0 |
| A | Phenoxyethanol | 0,5 |
| A | Disodium EDTA | 0,1 |
| A | Genencare^{®} OSMS BA (betaine) | 2,0 |
| A | Natura-Tec^{®} Rice Starch (Oryza sativa starch) | 2,0 |
| B | Sepimax^{®} Zen (polyacrylate crosspolymer-6) | 1,0 |
| B | Cetiol^{®} B (dibutyl adipate) | 3,0 |
| C | Massocare^{®} MCT (caprylic/capric triglyceride) | 2,0 |
| D | KSG-16 (dimethicone, dimethicone/vinyl dimethicone crosspolymer) | 3,0 |
| D | KF-56A (diphenylsiloxy phenyl trimethicone) | 5,0 |
| D | Cetiol^{®} Ultimate (undecane, tridecane, tocopherol, helianthus annuus seed oil) | 3,0 |
| E | Sepiplus^{™} 400 (Polyacrylate-13, Polyisobutene and Polysorbate 20) | 0,25 |
| F | Active ingredient: Example 1A, kojic acid or ascorbyl glucoside | 1,0 |
| | Total | 100.0 |

The hydrolysate of Example 1A, according to the present invention was compared with two well-known skin lightening components, namely, kojic acid and ascorbyl glucoside.

The tested compositions were prepared as follows. Phase A ingredients were weighed and mixed using a paddle mixer. Ingredients of phase B were dispersed and added to phase A until completely homogenized. Phase C ingredient was added and phase D ingredients were subsequently added one by one. Phase E ingredient was then added and the mixture was homogenized in a high-shear Ultra-Turrax mixer (at 3000rpm for 1 minute). Finally, the active ingredient F was added. The pH was checked to be in the range 5.5-6.5 (or otherwise adjusted with 10% citric acid or 10% NaOH).

For comparative purposes, a "base formulation", without any active ingredient, was prepared to be used as control composition. Thus, in this base formulation no active ingredient was added and its amount was supplied with water.

For Kojic acid and ascorbyl glucoside, their concentration in the tested composition was 1.0% (w/w). The hydrolysate of Example 1A was used at 100 ppm (0.01% w/w) in the composition, by previously dissolving it in water (10 mg/ml) and adding this solution in the tested formulation at 1% w/w.

Three different whitening compositions were tested, and compared to control. The results of the depigmentation assay are shown in the following table:

| **Tested compounds** | **Melanin decrease vs. control (µg)** | **SD** |
|---|---|---|
| Control | 0.00 | 0.70 |
| Kojic Acid 1% w/w | -6.50 | 0.21 |
| Ascorbyl glucoside 1% w/w | -5.92 | 0.11 |
| Example 1A 100 ppm | -7.61 | 0.48 |

The hydrolysate according to the invention (Example 1A) provided a reduction in melanin content in this assay, which is 17% higher than the reduction provided by kojic acid and 29% higher than the reduction provided by ascorbyl glucoside, even when applied at significantly lower concentration (0.01% *vs.* 1%).

### Example 4 Testing of the Bace2 inhibitory activity of the hydrolysate of Example 1

For assessing the inhibitory activity of the compound of Example 1 against Bace2, an *in vitro* assay was performed using SensoLyte^{®} 520 BACE2 Activity Assay Kit Fluorimetric (Anaspec), following the instructions of the manufacturer.

The results of this test showed that the hydrolysate of Example 1 inhibited the activity of BACE2 by 59%.

### Example 5 Composition comprising the hydrolysate of the invention

A composition according to the present invention was prepared using the components listed in the following table:

| **Ingredients** | | **Weight %** |
|---|---|---|
| A1 | Deionized water | q.s. 100% |
| A2 | Preservatives | 2.0 |
| | (Methylpropanediol, Caprylyl Glycol and phenylpropanol) | |
| A3 | Phenoxyethanol | 0.5 |
| A4 | Disodium EDTA | 0.1 |
| A5 | Anhydrous betaine extracted from sugar beet | 2.0 |
| A6 | Oryza Sativa starch | 2.0 |
| B1 | Polyacrylate crosspolymer-6 | 1.0 |
| B2 | Dibutyl adipate | 3.0 |
| B3 | Caprylic/capric Triglyceride | 2.0 |
| B4 | Dimethicone/Vinyl Dimethicone Crosspolymer and Dimethicone | 3.0 |
| B5 | Diphenylsiloxy phenyl trimethicone | 5.0 |
| B6 | Cyclopentasiloxane | 3.0 |
| C1 | SEPIPLUS^{™} 400 | 0.25 |
| | (Polyacrylate-13, Polyisobutene and Polysorbate 20) | |
| D1 | Aqueous solution of *M. communis* hydrolysate at 10 mg/mL | 1.0 (0.01) |
| | *(M. communis* hydrolysate content) | |
| E3 | Perfume | 0.2 |
| | Total | 100.0 |

For preparing the composition, components A1-A6 were mixed in a paddle stirrer until obtaining a homogeneous mixture ("phase A"). Components B1-B6 were separately mixed until obtaining a homogeneous mixture, and it was added to phase A under stirring for about 15 minutes. Ingredient C1 was added in a high-shear Ultra-Turrax homogenizer (about 2 minutes at 3000 rpm) and then in a paddle stirrer for about 15 minutes. Ingredient D1 was added to the composition under stirring. The pH of the mixture was checked to be in the range 5.5-6.5 (or otherwise adjusted with 10% citric acid or 10% NaOH). Finally, the component E3 was added.

The composition obtained was a white aqueous cream-gel.

### Example 6 Composition comprising the hydrolysate of the invention

A composition according to the present invention was prepared using the components listed in the following table:

| **Ingredients** | | **Weight %** |
|---|---|---|
| A1 | Emulium^{®} Mellifera MB | 5.0 |
| | (polyglyceryl-6 distearate, jojoba esters, polyglyceryl-3 beeswax and cetyl alcohol) | |
| A2 | Isohexadecane | 2.0 |
| A3 | Isopropyl isostearate | 1.0 |
| A4 | Isopropyl myristate | 2.0 |
| A5 | Tocopheryl acetate | 0.2 |
| B1 | Deionized water | q.s. |
| B2 | Potassium cetyl phosphate | 0.3 |
| B3 | Anhydrous betaine extracted from sugar beet | 2.0 |
| B4 | Preservatives | 2.0 |
| | (Methylpropanediol, Caprylyl Glycol and phenylpropanol) | |
| B5 | Phenoxyethanol | 0.5 |
| B6 | Disodium EDTA | 0.1 |
| B7 | Glycerin | 2.0 |
| B8 | Xanthan gum | 0.2 |
| C1 | SEPIPLUS^{™} 400 | 0.5 |
| | (Polyacrylate-13, Polyisobutene and Polysorbate 20) | |
| C2 | Cyclopentasiloxane | 2.0 |
| C3 | Dimethicone | 1.0 |
| C4 | Perfume | 0.3 |
| D1 | Aqueous solution of *M. communis* hydrolysate at 10 mg/mL | 1.0 (0.01) |
| | *(M. communis* hydrolysate content) | |
| | Total | 100.0 |

For preparing the composition, first components A1-A5 were heated to 70-75 °C and mixed to obtain a solution ("phase A"). Components B1-B8 were separately mixed to form a solution, which was heated to 70-75 °C. Phase A was added to this solution and was emulsified in a high-shear Ultra-Turrax mixer (at 3000rpm for 3 minutes, and then stirring at 300 rpm for about 10 minutes). The components C1-C3 were added at 60 °C, the mixture was allowed to cool down to room temperature and C4 was then added. Ingredient D1 was finally added to the previous mixture. The final pH of the composition was checked to be in the range 5.5-6.5 (or otherwise adjusted with 10% citric acid or 10% NaOH).

The product obtained was a white O/W emulsion.

## Claims

1. Process for preparing a *Myrtus communis* hydrolysate, wherein the process comprises the following steps:
a) extracting *Myrtus communis* material with an aqueous solvent;
b) enzymatically hydrolysing the extract obtained in step a) with a cellulase; and
c) enzymatically hydrolysing the hydrolysate obtained in step b) with a protease.

2. Process according to claim 1, wherein *Myrtus communis* material is selected from the leaves, the fruit and mixtures thereof, and preferably the *Myrtus communis* material are the leaves of *Myrtus communis.*

3. Process according to claims 1 or 2, wherein the aqueous solvent of step a) comprises at least 60% of water, preferably at least 70% of water, still more preferably at least 80% of water, still more preferably at least 90% of water, still more preferably at least 95% water, and still more preferably the aqueous solvent is water.

4. Process according to any one of claims 1 to 3, wherein the cellulase in step b) is an endo-cellulase (EC 3.2.1.4).

5. Process according to any one of claims 1 to 4, wherein the protease of step c) is selected from an endopeptidase, an exopeptidase, and mixtures thereof.

6. Process according to claim 5, wherein the endopeptidase is selected from a serine endopeptidase (EC 3.4.21), a metalloendopeptidase (EC 3.4.24), and mixtures thereof.

7. Process according to any one of claims 1 to 5, wherein the protease of step c) is a subtilisin (EC 3.4.21.62), and preferably is subtilisin A from *Bacillus licheniformis* (alcalase).

8. Process according to any one of claims 1 to 7, wherein after step c) the obtained hydrolysate is separated from insoluble material and collected as an aqueous solution.

9. Process according to claim 8, wherein the hydrolysate aqueous solution is dried to provide a dry hydrolysate.

10. *Myrtus communis* hydrolysate obtainable by the process according to any one of claims 1 to 9.

11. Cosmetic composition comprising the hydrolysate according to claim 10, optionally in combination with another cosmetic active ingredient, and at least one dermatologically acceptable carrier or vehicle, and wherein the cosmetic composition is preferably selected from the group consisting of a cream, gel, cream-gel, lotion, paste, foam, solution, suspension, emulsion, milk, and stick preparation.

12. Cosmetic composition according to claim 11, wherein the composition comprises from about 0.001 mg to about 5 mg, preferably from about 0.01 mg to about 1 mg, more preferably from about 0.02 mg to about 0.5 mg, and still more preferably about 0.1 mg of the hydrolysate according to claim 10 per g of composition.

13. Use of the hydrolysate according to claim 10 or of the cosmetic composition according to claims 11 or 12 for skin whitening.

14. Method for skin whitening comprising the step of topically applying a cosmetically effective amount of the hydrolysate according to claim 10 or of the composition according to claims 11 or 12 to a subject in need thereof.

15. Use according to claim 13 or method according to claim 14 for the elimination or reduction of hyperpigmented marks on the skin, the smoothing uneven skin tone, and/or the lightening of the skin tone.
